Europäisches Patentamt

European Patent Office

Office européen des brevets

(19)

(11) Veröffentlichungsnummer: **0 307 743 B1**

(12) # EUROPÄISCHE PATENTSCHRIFT

(45) Veröffentlichungstag der Patentschrift: **24.06.92**

(51) Int. Cl.⁵: **A61M 5/00**, A61M 1/00

(21) Anmeldenummer: **88114415.8**

(22) Anmeldetag: **03.09.88**

(54) **Absperrvorrichtung an einer Flüssigkeitsentnahme- oder Infusionseinrichtung.**

(30) Priorität: **17.09.87 DE 3731242**

(43) Veröffentlichungstag der Anmeldung:
**22.03.89 Patentblatt 89/12**

(45) Bekanntmachung des Hinweises auf die
Patenterteilung:
**24.06.92 Patentblatt 92/26**

(84) Benannte Vertragsstaaten:
**DE ES FR GB IT SE**

(56) Entgegenhaltungen:
**EP-A- 0 150 666**
**FR-A- 2 439 022**
**GB-A- 2 138 106**
**US-A- 3 920 215**

(73) Patentinhaber: **JOKA KATHETERTECHNIK
GMBH
Im Weiher 19
W-7450 Hechingen(DE)**

(72) Erfinder: **Sunnanväder, Lars
Ermelesstrasse 37
W-7450 Hechingen(DE)**
Erfinder: **Hartig, Thomas
Zu den Linden 14
W-7450 Hechingen-Bechtoldsweiler(DE)**

(74) Vertreter: **Weber, Dieter, Dr. et al
Dr. Dieter Weber und Dipl.-Phys. Klaus Seiffert Patentanwälte Gustav-Freytag-Strasse
25 Postfach 6145
W-6200 Wiesbaden 1(DE)**

EP 0 307 743 B1

## Beschreibung

Die Erfindung betrifft ein Absperrventil an einer Flüssigkeitsentnahme- oder Infusionseinrichtung mit einem büchsenförmigen Gehäuse mit einer seitlichen Öffnung zwischen seinen Enden, in deren Bereich ein an der Innenwand des Gehäuses dichtend anliegendes, elastisches Schlauchstück eingesetzt ist, und mit einem in die seitliche Öffnung radial beweglich eingesetzten Druckkörper, wobei eine rohrförmige Führungshülse, welche die seitliche Öffnung des Gehäuses umgibt, radial seitlich herausstehend am Gehäuse angebracht ist und der Druckkörper die Form eines Stiftes hat.

Es sind bereits Absperrvorrichtungen für Infusionseinrichtungen oder Blutentnahmeeinrichtungen bekannt, die mit Schläuchen oder Injektionsspritzen an einen gelegten Katheter angeschlossen werden können, um einem Patienten eine Infusionsflüssigkeit zuzuführen oder um Blut oder andere Körperflüssigkeiten abzunehmen.

Um ein Absperren eines solchen Anschlusses sowohl nach erfolgter Kathetisierung als auch bei gelegtem Katheter zu einem genauen Zeitpunkt zu ermöglichen, hat man bereits ein Kanülenanschlußstück mit einem büchsenförmigen Gehäuse ähnlich der eingangs beschriebenen Art entwickelt, wobei ein als Kugel ausgebildeter Druckkörper auf diametral bezüglich des Durchganges und auch des elastischen Schlauchstückes gegenüberliegenden Seiten angeordnet ist. Ein U-förmiger Schieber umgreift beide Kugeln und erlaubt in seiner einen Endstellung, daß das Schlauchstück ohne Zusammendrückung den Durchgang freiläßt, während die Kugeln in der anderen Endstellung des Schiebers aufeinanderzubewegt werden und den Durchgang dichtend verschließen.

Dieser bekannte Kanülenanschluß ist aber nicht nur kompliziert aufgebaut, sondern er hat vor allem die in Richtung der Kanüle längs verlaufende Bewegungsrichtung des Schiebers, wodurch der Benutzer dieser Absperrvorrichtung zumeist zwei Hände benötigt. Außerdem bringt die Bewegung und Betätigung in Richtung der Kanüle im Betrieb häufig Gefäßverletzungen mit sich, mindestens ist das Kathetisieren mit einer solchen Vorrichtung aber schmerzhaft.

Aus der GB-A-2 138 106 ist weiterhin ein Absperrventil an einer Flüssigkeitsentnahme- oder Infusionseinrichtung bekannt, bei der in einem büchsenförmigen Gehäuse zwei im Abstand voneinander ausgebildete Rohrabschnitte angeordnet sind. Die beiden Rohrabschnitte sind über ein elastisch verformbares Schlauchstück dichtend miteinander verbunden. Das büchsenförmige Gehäuse weist eine seitliche Öffnung auf, in die radial beweglich ein Druckkörper eingesetzt ist. Um das Absperrventil zu betätigen, wird der Druckkörper so gegen das elastische Schlauchstück gepreßt, daß es zusammengequetscht wird und somit die Strömungsmittelverbindung von dem einen Rohrstück zum anderen Rohrstück unterbrochen ist. Bei dieser Anordnung ist es als weniger vorteilhaft anzusehen, daß zum Absperren des Absperrventils der Druckkörper ständig gegen das Schlauchstück gedrückt werden muß, was beim Entnehmen von Blut oder Einspritzen einer Infusionslösung in die Blutbahn einer Person leicht zu Gefäßverletzungen und schmerzhaften Erscheinungen führen kann.

Der Erfindung liegt hingegen die Aufgabe zugrunde, eine Absperrvorrichtung der eingangs genannten Art zu schaffen, die einfach herzustellen und leicht zu betätigen ist, ohne daß schmerzhafte oder verletzende Tätigkeiten im Betrieb der Absperrvorrichtung auftreten.

Diese Aufgabe wird erfindungsgemäß dadurch gelöst, daß die Führungshülse mindestens ein Paar von Sperranschlägen aufweist, und daß an dem, dem Schlauchstück abgewandten Ende des Stiftes eine Kappe mit einem die Führungshülse wenigstens teilweise übergreifenden, elastischen Mantel und Schnappring befestigt ist, wobei der Schnappring so gestaltet ist, daß er über die Sperranschläge übergreifen kann, und in dieser Stellung die Kappe so arretiert wird, daß der Druckkörper das Schlauchstück dichtend schließt.

Durch die seitlich radial vom büchsenförmigen Gehäuse herausstehende Führungshülse in Rohrform, in welcher der Druckkörper geführt wird, welcher die Form eines Stiftes hat, wird die Bewegungsrichtung bei der Betätigung des neuen Absperrventils quer zur Längsrichtung der Kanüle eingestellt, wodurch sich mehrere Vorteile ergeben. Nicht nur, daß beim Betätigen durch den Arzt oder die Krankenschwester Schmerzeinwirkungen vermieden werden können und die Gefäße nicht mehr verletzt werden, sondern die Daumenbewegung läßt sich wesentlich besser mit der seitlichen Bewegungsrichtung radial zum büchsenförmigen Gehäuse koordinieren. Außerdem kann das Pflegepersonal die Bedienung mit nur einer Hand ausführen, so daß die andere Hand für weitere Tätigkeiten frei bleibt. Dadurch wird auch die Ansteckungsgefahr des Pflegepersonals bei der Krankenbehandlung herabgesetzt.

Je nach dem Druck auf den als Stift ausgebildeten Druckkörper quetscht dieser das quer zu seiner Bewegungsrichtung liegende Schlauchstück mehr oder weniger zusammen, so daß der Durchlaufkanal entweder gedrosselt oder vollständig dichtend geschlossen wird. Der Schnappring übergreift die Sperranschläge und ermöglicht ohne weitere Betätigung eine Arretierung des Druckkörpers in einer bestimmten Position, ohne daß herstellungstechnisch komplizierte Teile vorgesehen sein müßten. Die Kappe am sogenannten oberen Ende des Druckstiftes hat die Gestalt einer Scheibe, deren Ebene quer zur Längsrichtung des Druckstiftes liegt, und am Umfang dieser Scheibe ist ein elastischer Mantel angebracht, der sich mithin in gleicher Richtung erstreckt wie der Druckstift und dabei einen Zylindermantel im Abstand vom Außenumfang des Druckstiftes aufspannt. Dieser elastische Mantel ist zwar in der Mitte "unten", d.h. auf der dem büchsenförmigen Gehäuse zugewandten Seite hin offen, an diesem unteren Ende weist der Mantel aber einen schmalen Bund oder Ring auf, welcher als Schnappring ausgestaltet ist. Der Innendurchmesser des Schnappringes ist so bemessen, daß er im Abstand von der Führungshülse verschoben werden kann und dabei mit den Sperranschlägen in Eingriff kommen kann. Zwei zu einem Paar zusammengefaßte Sperranschläge beaufschlagen somit den Mantel, die Kappe und den Druckkörper mittensymmetrisch, so daß sich der Druckstift nicht verkantet. Ist der Schnappring beispielsweise über einen oder beide Sperranschläge beim Betätigen und Hinunterschieben hinübergefahren, dann kann man diese Arretierung wieder dadurch lösen, daß man den Mantel zwischen zwei Fingern zusammendrückt, wodurch er in Draufsicht ovalisiert wird. Befinden sich dann die Sperranschläge im Bereich der größeren Achse dieses Ovals, dann versteht sich, daß man den Schnappring unter Aufhebung der Arretierung über die Sperranschläge ohne weiteres hinüberheben oder -schieben kann, um beispielsweise den Druckstift wieder nach außen oben vom Schlauchstück fortzuschieben und den Durchgang für die Infusionslösung oder Körperflüssigkeit freizugeben.

Der Durchlaufkanal für die Körperflüssigkeiten in dem büchsenförmigen Gehäuse wird durch den Druckkörper gemäß der Erfindung nicht nur vollständig verschlossen, sondern man kann ihn auch teilweise verschließen. Zum Beispiel wird bei besonderen Anwendungsarten für eine Infusionseinrichtung eine Stahlkanüle durch das Schlauchstück und in ein Katheterrohr am Gehäuse geschoben. Bei Betätigen des Druckkörpers durch seitliches Drücken mit dem Daumen auf die Kappe wird das untere oder vordere Ende des Druckstiftes so gegen das Schlauchstück gepreßt, daß sich dieses dichtend um die Stahlkanüle herumlegt. Diesen Zwischenzustand kann man beispielsweise durch leichtes Drücken auf den Druckkörper erreichen. Währenddessen kann dann die Stahlkanüle herausgezogen werden, ohne daß Körperflüssigkeit am Schlauchstück vorbei nach außen gelangen könnte. Während des leichten Druckes auf den Druckkörper wird also die Stahlkanüle oder Nadel herausgezogen. Das Schlauchstück fügt sich an die Oberfläche der Stahlkanüle oder Nadel an und dichtet diese so ab, daß beispielsweise kein Blut zwischen der Nadel und dem Schlauchstück herausspritzen kann. Nach vollständigem Herausziehen der Nadel oder Kanüle dichtet das Schlauchstück dann den gesamten Durchgang vollständig ab, wenn der leichte Druck auf den Druckkörper fortgesetzt oder gegen Ende etwas verstärkt wird.

Es versteht sich, daß eine solche Absperrvorrichtung als Verschließventil auch an Einspritzeinrichtungen angebaut werden kann.

Bei vorteilhafter Weiterbildung der Erfindung hat der Schnappring eine sich in Richtung des Gehäuses erweiternde kegelstumpfförmige Fläche. Es ist zweckmäßig, daß der stiftförmige Druckkörper selbst fest oder starr ist, um den Druck für das Zusammenquetschen des Schlauchstückes gut vom Daumen auf das Schlauchstück übertragen zu können. Andererseits wünscht man einen elastischen Mantel, damit dieser eindrückbar ist, um herstellungstechnisch einfache Werkzeuge und Produkte zu ermöglichen. Nicht nur der elastische Mantel sondern auch der Schnappring, die gegebenenfalls aus dem gleichen Kunststoff bestehen können, sollte daher elastisch biegbar und eindrückbar sein. Durch eine verjüngt zulaufende Schrägfläche im Bereich des Schnappringes wird mit Unterstützung der Weichheit des Materials ein gutes Hinüberschieben oder Hinübergleiten des Schnappringes über den oder die Sperranschläge gewährleistet.

Im vorstehenden Sinne der Vereinfachung der Herstellung und Schaffung eines preiswerten Druckkörpers ist es erfindungsgemäß ferner zweckmäßig, wenn der Druckkörper, die Kappe mit Mantel und der Schnappring einstückig aus Kunststoff geformt sind. Der stiftförmige Druckkörper selbst erhält seine Festigkeit und Steifigkeit durch die Größe seines Durchmessers, d.h. die Dicke des massiv eingesetzten Materials für den Druckkörper. Die angeformte Kappe auf der oberen oder äußeren Seite des Druckstiftes und erst recht der am Außenumfang der scheibenförmigen Kappe angeformte elastische Mantel besteht aus weniger Material in Gestalt dünnerer Wandungen und ist deshalb ohne Kraftaufwand eindrückbar. Obwohl der Schnappring eine gewisse Weichheit hat, kann er dennoch durch seine Formgebung mit einfachsten Mitteln in Arretiereingriff mit dem Sperranschlag kommen.

Zweckmäßig ist es gemäß der Erfindung ferner, wenn die Sperranschläge eines Paares am Außenumfang der Führungshülse, einander diametral gegenüberliegend, im gleichen Höhenabstand vom Gehäuse und ringteilförmig radial nach außen vorstehend angebracht sind. Die symmetrische Beaufschlagung des Druckkörpers mit der Eingriffskraft zwischen Sperranschlag und Schnappring beim Herunterdrücken des Druckkörpers wurde vorstehend schon angesprochen. Durch die diametral gegenüberliegende Ausgestaltung der zwei, zu einem Paar zusammengefaßten Sperranschläge begünstigt die Führung des Druckstiftes in der Führungshülse. Die Form des jeweils radial nach außen, d.h. seitlich zur Längsrichtung der Führungshülse herausstehenden Sperranschläge ist in Draufsicht, wenn man in Richtung der Achse der Führungshülse blickt, wie ein Ringteil. Vorzugsweise ist das eine Ringteil ebenso lang wie das diametral gegenüberliegende Ringteil und in radialer Richtung mit einer solchen Ausdehnung versehen, daß die Außenkante des ringteilförmigen Sperranschlages fast bündig mit dem elastischen Mantel liegt. Es ist dabei bevorzugt, den elastischen Mantel mit diametral gegenüberliegenden Öffnungen oder Schlitzen zu versehen, durch welche die Sperranschläge hindurchragen. Auf diese Weise werden der elastische Mantel und der Druckkörper zugleich auch mittels der Sperranschläge geführt.

In diesem Zusammenhang ist es vorteilhaft, wenn erfindungsgemäß zwei Paare von Sperranschlägen im Winkelabstand und im Höhenabstand zueinander vorgesehen sind. Als Höhenabstand ist der Abstand in Längsrichtung der Führungshülse verstanden, also senkrecht zur Längsrichtung des büchsenförmigen Gehäuses. Erfindungsgemäß sind also ein dichter am Gehäuse sitzendes, unteres Paar von Sperranschlägen und ein oberes oder äußeres Paar von Sperranschlägen vorgesehen. Beide Paare sind im Höhenabstand zueinander angeordnet und außerdem winkelig versetzt. Blickt man in Achsrichtung der Führungshülse, dann liegt die Verbindungslinie eines Paares von Sperranschlägen beispielsweise im rechten Winkel zur Verbindungslinie des zweiten Paares von Sperranschlägen. Ist der Ringteil des jeweiligen Sperranschlages, in Achsrichtung der Führungshülse gesehen, klein genug, beispielsweise innerhalb eines Winkels von 45°, dann kann bei entsprechend groß bemessenen Durchbrüchen, Öffnungen oder Schlitzen im elastischen Mantel der Druckkörper von beiden Paaren von Sperranschlägen geführt sein. Der Mantel ist dann zu einer Anordnung von vier am Umfang im Abstand zueinander angeordneten Säulen entartet, erfüllt aber nach wie vor die Funktion des elastischen Mantels, d.h. ist eindrückbar, wenn man von außen auf diese Säulen drückt, ist damit ovalisierbar und erlaubt das Aufheben der Arretierposition.

Weiterhin ist es erfindungsgemäß von Vorteil, wenn der Höhenabstand der zwei Paare von Sperranschlägen voneinander größer als der Deformationsweg des Schlauchstückes ist. Hierdurch wird erreicht, daß das Schlauchstück nicht nur teilweise sondern vollständig zusammengedrückt wird, wenn der Druckkörper seinen vollen Hub durchlaufen hat.

Man kann das untere oder dichter am büchsenförmigen Gehäuse angebrachte Paar von Sperranschlägen ebenfalls mit einer kegelstumpfförmigen Außenoberfläche derart versehen, daß die Spitze des Kegels oben außen zu liegen kommt, der Kegelstumpf sich also in Richtung auf den Schnappring verjüngt. Dies kommt der Gleitbewegung des Schnappringes entgegen. Der Benutzer kann auf diese Weise den Schnappring leichter über das untere Paar von Sperranschlägen hinüberschieben. Umgekehrt wird diese Arretierposition (z.B. vollständiges Verschließen des Schlauchstückes) durch Ovalisieren des Mantels aufgehoben werden.

Das äußere obere Paar von Sperranschlägen, welches um 90° zu den unteren inneren Sperranschlägen versetzt angeordnet ist, weist nicht eine solche Abschrägung auf. Es handelt sich hier um die Sperranschläge, die ein Abheben oder Herausziehen des Druckkörpers aus der Absperrvorrichtung verhindern sollen.

Eine andere Ausführungsform der Erfindung ist dadurch gekennzeichnet, daß auf der Innenfläche des elastischen Mantels an der Kappe ein Gewinde angebracht ist und wenigstens eine Wulst oder ein Außengewinde an der Führungshülse vorgesehen ist. Das Gewinde im Mantel erlaubt ein Zusammenwirken mit einem Außengewinde auf der Führungshülse. Diese Ausführungsform hat den Vorteil, daß der Durchgang für die Körperflüssigkeit im Schlauchstück nicht unbeabsichtigt durch einen Druck oder Stoß von oben auf den Druckkörper abgequetscht wird. Für das Drosseln oder vollständige Abquetschen und Verschließen des Schlauchstückes ist bei dieser Ausführungsform eine Drehung bewußt erforderlich. Die Schraubenbewegung erlaubt ein besonders sorgfältiges und exaktes Einstellen des Druckkörpers und damit Drosseln des Durchganges im Schlauchstück.

Vorteilhaft ist es für alle Ausführungsformen, wenn der Druckkörper mit Gleitberührung in der Führungshülse geführt ist. Hierdurch wird ein Verkanten beim Betätigen des Druckstiftes vermieden. Vorzugsweise ist die Gleitberührung sowohl am äußeren oberen als auch unteren inneren Ende der Führungshülse vorgesehen.

Weiterhin ist die Erfindung dadurch ausgestaltet, daß der Innendurchmesser der Führungshülse größer ist als der Außendurchmesser der seitlichen Öffnung im Gehäuse und der Druckkörper an seinem freien, dem Schlauchstück zugewandten Ende verjüngt ausgestaltet ist. Die Ausgestaltung einer Führungshülse und der Sperranschläge ist günstiger bei etwas größeren Abmessungen. Das freie, innere oder untere Ende des Druckstiftes andererseits sollte nicht zu groß sein im Verhältnis zum Außendurchmesser des Schlauchstückes. Beim Hereindrücken des Druckkörpers steht nämlich nur soviel Volumen für das Material des Schlauchstückes zur Verfügung, wie Durchgang unter dem Druckkörper vorhanden war. Bei kleinerem Durchmesser des Druckkörpers an seinem unteren freien Ende im Verhältnis zu seinem oberen äußeren Ende erreicht man ein gutes Verteilen oder Herumschieben des Schlauchmaterials beim Zusammendrücken des Schlauchstückes. Damit benötigt man für das Eindrücken weniger Kraft und braucht das Material des Schlauchstückes nicht unter großer Beanspruchung zur Seite zu quetschen.

Weitere Vorteile, Merkmale und Anwendungsmöglichkeiten der vorliegenden Erfindung ergeben sich aus der folgenden Beschreibung bevorzugter Ausführungsbeispiele in Verbindung mit den Zeichnungen. Es zeigen:

Figur 1 abgebrochen den Längsschnitt durch eine Absperrvorrichtung gemäß der Erfindung mit offenem Durchgang,

Figur 2 eine Schnittansicht entlang der Linie II-II der Figur 1,

Figur 3 eine Querschnittsansicht entlang der Linie III-III der Figur 1,

Figur 4 eine ähnliche Querschnittsansicht, jedoch entlang der Linie IV-IV der Figur 5 mit zusammengequetscht gezeigtem Schlauchstück,

Figur 5 den Längsschnitt ähnlich wie Figur 1, wobei jedoch der Druckkörper nach unten innen geschoben und das Schlauchstück zusammengequetscht ist und

Figur 6 eine Schnittansicht entlang der Linie VI-VI der Figur 5.

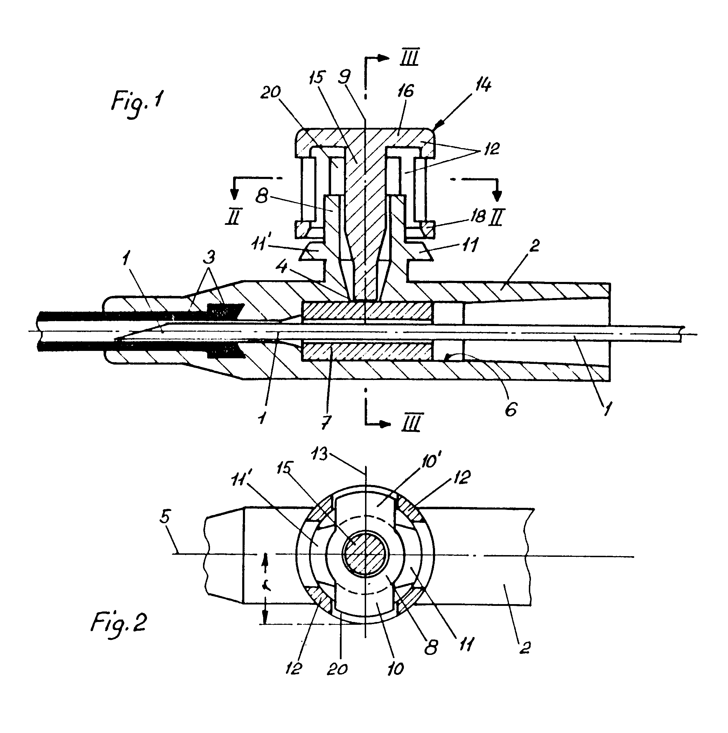

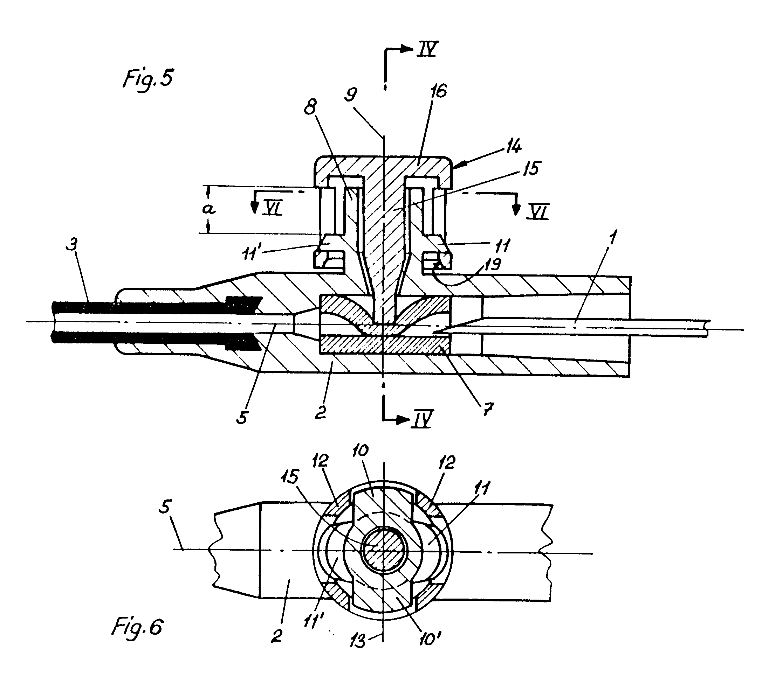

Die Absperrvorrichtung ist hier an einer Infusionskanüle gezeigt, wobei man die Stahlkanüle 1 teilweise in das büchsenförmige Gehäuse 2 eingeschoben sieht. Von der hinteren oder Bedienungsseite (in den Figuren 1 und 5 rechts) her ist das Gehäuse 2 mit einer nicht näher bezeichneten Anschlußhülse versehen, die innen leicht konisch ist und dem Anschluß an eine Injektionsspritze, einen Schlauch oder dergleichen dient. Auf der gegenüberliegenden oder vorderen Seite des Gehäuses 1 sieht man das eingesteckte Katheterrohr 3, in welches die Stahlkanüle 1 eingeschoben wird.

Das Gehäuse 2 ist in den Darstellungen oben mit einer seitlichen Öffnung 4 zwischen seinen Enden versehen. Unter dieser Öffnung 4 und besser im Bereich derselben, nämlich in Längsrichtung 5 des Gehäuses 2 nach vorn und nach hinten ist anliegend an der Innenwand 6 ein Schlauchstück 7 eingesetzt, welches aus elastischem Material hergestellt ist, z.B. Silicon. Dieses Schlauchstück 7 ist leicht biegsam und dichtet gegen die Innenwand 6 des Gehäuses 2 ab. Diese Abdichtung erfolgt bei der Darstellung der Figur 1 bis auf die Fläche der Öffnung 4 im gesamten Bereich der Außenfläche des Schlauchstückes 7, im Zustand der Figur 5 hingegen nur an den Enden des Schlauchstückes, weil dieses in der Mitte zusammengedrückt ist. Man erkennt, wie im offenen Zustand des Durchganges die Stahlkanüle 1 auch durch das Schlauchstück 7 hindurchgeführt ist.

Die seitliche Öffnung 4 des Gehäuses 2 umgebend ist eine rohrförmige Führungshülse 8 aus demselben Kunststoff wie das Gehäuse 2 an diesem angeformt, vorzugsweise Polyethylen. Die Längsachse 9 der Führungshülse 8 liegt ersichtlich senkrecht zur Längsachse 5 des Gehäuses 2. Deshalb wird die Führungshülse 8 als radial nach außen seitlich vom Gehäuse 2 herausstehend angesehen.

Zwei Paare von Sperranschlägen 10, 10', 11, 11' sind ringteilförmig von der Führungshülse 8 radial nach außen vorstehend angeformt. Bei jedem Paar liegen sich die zwei dieses Paar bildenden Sperranschläge 10, 10', 11, 11' einander diametral gegenüber. Sie schließen sich an den Außenumfang der Führungshülse 8 an und enden in einem Abstand von der Mittelachse 9 der Führungshülse 8, der etwas kleiner als der Radius r des später noch zu beschreibenden Mantels 12 ist. Es gibt ein unteres Paar von Sperranschlägen 11, 11', die näher am Gehäuse 2 liegen als das obere oder äußere Paar von Sperranschlägen 10, 10', die in einem Höhenabstand a vom unteren oder inneren Paar von Sperranschlägen 11, 11' angeordnet sind. Aus den Figuren 2 und 6, d.h. mit Blickrichtung in Achsrichtung 9 der Führungshülse 8 erkennt man, daß die Verbindungslinie der zwei Sperranschläge 11, 11', die mit der Längsrichtung 5 zusammenfällt, senkrecht zur Verbindungslinie 13 der Sperranschläge 10, 10' verläuft, die beiden Paare von Sperranschlägen also in einem Winkelabstand von 90° zueinander angeordnet sind.

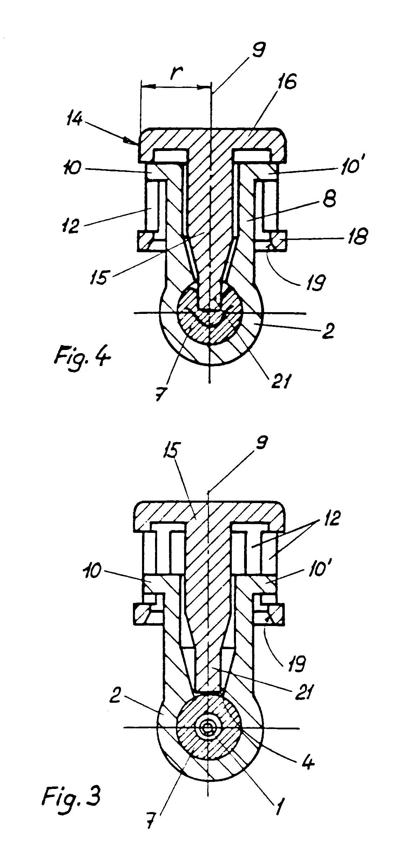

Während das obere Paar von Sperranschlägen 10, 10', die in den Figuren 3 und 4 besonders deutlich zu sehen sind, gerades Profil haben, ist die radial äußere Fläche der unteren Sperranschläge 11, 11', wie man am besten aus den Figuren 1 und 5 sieht, kegelstumpfförmig verjüngt derart ausgestaltet, daß die Kegelstumpfspitze auf der Achslinie 9 außerhalb der Führungshülse 8 liegt, d.h. der Kegelstumpf sich nach außen oben hin verjüngt.

Der allgemein mit 14 bezeichnete Druckkörper besteht in seinem Wesen aus dem stiftförmigen Teil 15, der an seinem äußeren oberen Ende eine Kappe 16 und den elastischen Mantel 12, an seinem unteren, inneren Ende den Schnappring 18 angeformt hat. Dieser hat eine sich in Richtung des Gehäuses 2 erweiternde, kegelstumpfförmige Fläche 19, welche also zu der oben beschriebenen kegelstumpfförmigen Fläche der Sperranschläge 11, 11' paßt.

Wegen der Länge der Sperranschläge in radialer Richtung (etwas kleiner als der Radius r des Außendurchmessers des Mantels 12) sind im elastischen Mantel 12 längliche Öffnungen oder Schlitze 20 gebildet, zwischen denen säulenartig der Mantel 12 stehen bleibt.

Aus der jeweiligen Längsschnittansicht des Druckstiftes 15 erkennt man, daß sowohl dieser als auch die ihn umgebende Führungshülse 8 im oberen äußeren Bereich einen größeren Durchmesser als im unteren inneren Bereich neben dem Schlauchstück 7 hat. Dies bedeutet, daß der Druckstift 15 in seinem unteren inneren Drittel als verjüngter Stift 21 ausgestaltet ist. Die tatsächliche Druckfläche ist also etwa so groß wie die Fläche der seitlichen Öffnung 4 im Gehäuse 2.

Beim Herausziehen der Stahlkanüle 1 beginnt etwa im Zustand der Figur 1 die Tätigkeit für das Pflegepersonal, durch leichten Druck von oben auf den Druckkörper 14, den Ringraum Zwischen Stahlkanüle 1 und Schlauchstück 7 abdichtend zu schließen. Mit anderen Worten bewegt sich der Druckstift 15 nach innen unten in Richtung auf das Schlauchstück 7 und quetscht dieses teilweise unter leichtem Druck zusammen, während die Stahlkanüle 1 noch herausgezogen wird.

Dann ist fast der Zustand der Figuren 4 bis 6 erreicht, wobei die letzte Bewegung bis zum vollständigen Hineindrücken des verjüngten Stiftes 21 in das Volumen im Durchgang dann erfolgt, wenn gerade die abgeschrägte Spitze der Stahlkanüle 1 aus dem Schlauchstück 7 herausgezogen wird. Jetzt ist der Zustand der Figuren 4 bis 6 erreicht. Der eigentliche zylinderförmige, mit kleinerem Außendurchmesser versehene Druckstift 21 befindet sich in seiner Endstellung. Gemäß Darstellung der Figur 4 kann sich das Siliconmaterial des Schlauchstückes 7 gut um die Außenflächen des Stiftteils 21 herumlegen und verteilen. Der eigentliche Durchgang im Schlauchstück 7 ist im Zustand der Figur 4 zu einer U-förmigen Linie zusammengefallen. Die Abdichtung ist vollständig, das Sperrventil hat seine abdichtende Endposition erreicht.

Bei dieser Position ist der Schnappring 18 über die unteren inneren Sperranschläge 11, 11′ hinübergeschoben worden und unter diese eingeschnappt. Dadurch ist eine Arretierposition erreicht, die erst wieder aufgehoben werden kann, wenn man gemäß Darstellung der Figur 6 von oben und unten drückt, den Mantel 12 also derart ovalisiert, daß dessen größere Achse in Richtung der Längsachse 5 der Kanüle liegt. Dann erst können die Schnappringteile über die Sperranschläge 11, 11′ wieder nach oben gezogen werden. Die elastische Rückstellkraft des Schlauchstückes 7 hilft mit, die Schnappringteile nach oben zu drücken.

Nach oben hin ist ein Hinüberziehen über die äußeren oberen Sperranschläge 10, 10′ nicht erwünscht. Man sieht aus der Position der Figur 3, daß dies die Endstellung ist. Würde der Schnappring 18 auch über die äußeren Sperranschläge 10, 10′ hinausgeführt werden, dann würde auf diese Weise der Druckkörper 14 von der gesamten Absperrvorrichtung separiert. Dies ist aber für den normalen Betrieb unerwünscht.

Jederzeit während der Behandlung kann der Absperrvorgang wiederholt werden.

**Patentansprüche**

1.  Absperrventil an einer Flüssigkeitsentnahme- oder Infusionseinrichtung mit einem büchsenförmigen Gehäuse (2) mit einer seitlichen Öffnung (4) zwischen seinen Enden, in deren Bereich ein an der Innenwand (6) des Gehäuses (2) dichtend anliegendes, elastisches Schlauchstück (7) eingesetzt ist, und mit einem in die seitliche Öffnung (4) radial beweglich eingesetzten Druckkörper (14), wobei eine rohrförmige Führungshülse (8), welche die seitliche Öffnung (4) des Gehäuses (2) umgibt, radial seitlich herausstehend am Gehäuse (2) angebracht ist und der Druckkörper (14) die Form eines Stiftes (15) hat, dadurch gekennzeichnet, daß die Führungshülse (8) mindestens ein Paar von Sperranschlägen (10, 10; 11, 11) aufweist, und daß an dem, dem Schlauchstück (7) abgewandten Ende des Stiftes (15) eine Kappe (16) mit einem die Führungshülse (8) wenigstens teilweise übergreifenden, elastischen Mantel (12) und Schnappring (18) befestigt ist, wobei der Schnappring (18) so gestaltet ist, daß er über die Sperranschläge (10, 10'; 11, 11') übergreifen kann, und in dieser Stellung die Kappe (16) so arretiert wird, daß der Druckkörper (14) das Schlauchstück (7) dichtend schließt.

2.  Vorrichtung nach Anspruch 1, dadurch gekennzeichnet, daß der Schnappring (18) eine sich in Richtung des Gehäuses (2) erweiternde kegelstumpfförmige Fläche (19) hat.

3.  Vorrichtung nach Anspruch 1 oder 2, dadurch gekennzeichnet, daß der Druckkörper (14), die Kappe (16) mit Mantel (12) und Schnappring (18) einstückig aus Kunststoff geformt sind.

4.  Vorrichtung nach einem der Ansprüche 1 bis 3, dadurch gekennzeichnet, daß die Sperranschläge (10, 10′; 11, 11′) eines Paares am Außenumfang der Führungshülse (8), einander diametral gegenüberliegend, im gleichen Höhenabstand vom Gehäuse (2) und ringteilförmig radial nach außen vorstehend angebracht sind.

5.  Vorrichtung nach einem der Ansprüche 1 bis 4, dadurch gekennzeichnet, daß zwei Paare von Sperranschlägen (10, 10′; 11, 11′) im Winkelabstand und im Höhenabstand (a) zueinander vorgesehen sind.

6. Vorrichtung nach einem der Ansprüche 1 bis 5, dadurch gekennzeichnet, daß der Höhenabstand (a) der zwei Paare von Sperranschlägen (10, 10′; 11, 11′) voneinander größer als der Deformationsweg des Schlauchstückes (7) ist.

7. Vorrichtung mit einem Gehäuse (2), dadurch gekennzeichnet, daß auf der Innenfläche des elastischen Mantels (12) an der Kappe (16) ein Gewinde angebracht ist und wenigstens eine Wulst oder ein Außengewinde an der Führungshülse (8) vorgesehen ist.

8. Vorrichtung nach einem der Ansprüche 1 bis 7, dadurch gekennzeichnet, daß der Druckkörper (14) mit Gleitberührung in der Führungshülse (8) geführt ist.

9. Vorrichtung nach einem der Ansprüche 1 bis 8, dadurch gekennzeichnet, daß der Innendurchmesser der Führungshülse (8) größer ist als der Durchmesser der seitlichen Öffnung (4) im Gehäuse (2) und der Druckkörper (14) an seinem freien, dem Schlauchstück (7) zugewandten Ende verjüngt (21) ausgestaltet ist.

## Claims

1. A shut-off valve on a liquid drainage or infusion device comprising a sleeve-shaped housing (2) having a lateral opening (4) between its ends, in the region of which is inserted an elastic tube portion (7) which bears sealingly against the inside wall (6) of the housing (2), and a pressing body (14) which is radially movably fitted into the lateral opening (4), wherein a tubular guide sleeve (8) which embraces the lateral opening (4) of the housing (2) is mounted on the housing (2) in radially laterally projecting relationship and the pressing body (14) is in the form of a pin (15) characterised in that the guide sleeve (8) has at least one pair of locking abutments (10, 10'; 11, 11') and that fixed to the end of the pin (15) which is remote from the tube portion (7) is a cap (16) with a resilient peripheral portion (12) which at least partially engages over the guide sleeve (8), and a snap ring (18), the snap ring (18) being of such a configuration that it can engage over the locking abutments (10, 10'; 11, 11') and in that position the cap (16) is so arrested that the pressing body (14) sealingly closes the tube portion (7).

2. Apparatus according to claim 1 characterised in that the snap ring (18) has a frustoconical surface (19) which enlarges in the direction of the housing (2).

3. Apparatus according to claim 1 or claim 2 characterised in that the pressing body (14) and the cap (16) with peripheral portion (12) and snap ring (18) are formed integrally from plastics material.

4. Apparatus according to one of claims 1 to 3 characterised in that the locking abutments (10, 10'; 11, 11') of a pair are disposed at the outside periphery of the guide sleeve (8), in mutually diametrally opposite relationship, at the same spacing in respect of height from the housing (2) and in such a way as to project radially outwardly in the form of ring portions.

5. Apparatus according to one of claims 1 to 4 characterised in that there are provided two pairs of locking abutments (10, 10'; 11, 11') at an angular spacing and at a spacing in respect of height (a) relative to each other.

6. Apparatus according to one of claims 1 to 5 characterised in that the spacing in respect of height (a) of the two pairs of locking abutments (10, 10'; 11, 11') from each other is greater than the deformation travel of the tube portion (7).

7. Apparatus having a housing (2) characterised in that a screwthread is provided on the inside surface of the elastic peripheral portion (12) on the cap (16) and at least one bead or an external screwthread is provided on the guide sleeve (8).

8. Apparatus according to one of claims 1 to 7 characterised In that the pressing body (14) is guided with sliding contact in the guide sleeve (8).

9. Apparatus according to one of claims 1 to 8 characterised in that the inside diameter of the guide sleeve (8) is larger than the diameter of the lateral opening (4) in the housing (2) and the pressing body (14) is of a reduced configuration (21) at its free end towards the tube portion (7).

## Revendications

1. Robinet d'arrêt installé sur un dispositif de prélèvement ou de perfusion de liquides, comportant un boîtier (2) en forme de manchon, comportant un orifice latéral (4) entre ses extrémités, dans la zone desquelles est installé un morceau de tuyau flexible élastique (7), qui s'appuie d'une manière étanche contre la paroi intérieure (6) du boîtier (2), et comportant un poussoir (14), disposé dans l'orifice latéral (4) de façon à pouvoir subir un déplacement radial, où une douille de guidage tubulaire (8), qui entoure l'orifice latéral (4) du boîtier (2), est disposée contre le boîtier (2) de façon à faire saillie radialement vers le côté, et le poussoir (14) a la forme d'une broche (15), caractérisé en ce que la douille de guidage ( 8) comporte au moins une paire de butées d'arrêt (10, 10 ; 11, 11), et qu'un chapeau (16), comportant une surface latérale élastique, recouvrant au moins partiellement la douille de guidage, ainsi qu'une bague à déclic (18), est fixé à l'extrémité de la broche (15) opposée au morceau de tuyau flexible (7), la bague à déclic (18) ayant une configuration telle qu'elle puisse recouvrir les butées d'arrêt (10, 10' ; 11, 11'), et que, dans cette position, le chapeau (16) soit bloqué de façon que le poussoir (14) obture d'une manière étanche le morceau de tuyau flexible (7).

2. Dispositif selon la revendication 1, caractérisé en ce que la bague à déclic (18) a une surface tronconique (19), qui s'évase dans la direction du boîtier (2).

3. Dispositif selon la revendication 1 ou 2, caractérisé en ce que le poussoir (14), le chapeau (16) avec sa surface latérale (12) et la bague à déclic (18) sont moulés en une seule pièce à partir d'une matière plastique.

4. Dispositif selon l'une des revendications 1 à 3, caractérisé en ce que les butées d'arrêt (10, 10' ; 11, 11') d'une paire sont disposées, sur la périphérie extérieure de la douille de guidage (8), en étant diamétralement opposées l'une à l'autre, à la même distance en hauteur par rapport au boîtier (2), sous la forme d'un segment annulaire, en dépassant radialement vers l'extérieur.

5. Dispositif selon l'une des revendications 1 à 4, caractérisé en ce qu'on prévoit deux paires de butées d'arrêt (10, 10' ; 11, 11'), faisant l'une par rapport à l'autre une certaine distance angulaire et une certaine distance (a) en hauteur.

6. Dispositif selon l'une des revendications 1 à 5, caractérisé en ce que la distance (a) en hauteur entre les deux paires de butées d'arrêt (10, 10' ; 11, 11') l'une par rapport à l'autre est supérieure à la déformation du morceau de tuyau flexible (7).

7. Dispositif comportant un boîtier (2), caractérisé en ce que, sur la surface intérieure de la surface latérale élastique (12), un filetage est rapporté au chapeau (16), et qu'on prévoit au moins un bourrelet ou un filetage extérieur sur la douille de guidage (8).

8. Dispositif selon l'une des revendications 1 à 7, caractérisé en ce que le poussoir (14) est guidé par contact de glissement dans la douille de guidage (8).

9. Dispositif selon l'une des revendications 1 à 8, caractérisé en ce que le diamètre intérieur de la douille de guidage (8) est supérieur au diamètre de l'orifice latéral (4) du boîtier (2), et que le poussoir (14), en son extrémité libre dirigée vers le morceau de tuyau flexible (7), présente une partie rétrécie (21).

Fig.1

Fig.2

Fig. 4

Fig. 3

Fig.5

Fig.6